# EUROPEAN PATENT SPECIFICATION

(11) **EP 2 846 472 B1**
(45) Date of publication and mention of the grant of the patent: **28.08.2019**
(21) Application number: 14186526.1
(22) Date of filing: 25.10.2011
(51) Int. Cl.: H04W 4/80, G16H 10/60, G16H 40/67, A61B 5/00, G06Q 10/00

(54) **METHOD AND SYSTEM OF COMMUNICATING DATA IN A NEAR FIELD COMMUNICATION ENVIRONMENT**
Verfahren und System zur Übermittlung von Daten in einer Nahfeldkommunikationsumgebung
Procédé et système de communication de données dans un environnement de communication en champ proche

(30) Priority: 25.10.2010 IN CH31732010
(43) Date of publication of application: 11.03.2015
(62) Divisional of application: 11836593.1
(73) Proprietor: Samsung Electronics Co., Ltd., Gyeonggi-do 443-742 (KR)
(72) Inventor: Reddy Badvel, Jayabharath, IN-560093 Bangalore (IN); Arunan, Thenmozhi, IN-560093 Bangalore (IN); Won, Eun-Tae, 443-742 Gyeonggi-do (KR)
(74) Representative: Nederlandsch Octrooibureau

(56) References cited:
- EP-A2- 2 573 970
- WO-A1-2009/068931
- WO-A1-2010/077194
- US-A1- 2008 162 312
- US-A1- 2008 252 415
- US-A1- 2009 147 803
- US-A1- 2009 248 437
- US-A1- 2010 045 425
- "Android NDEF Push Protocol Specification", , 22 February 2011 (2011-02-22), XP055163343, Retrieved from the Internet: URL:http://static.googleusercontent.com/ex ternal_content/untrusted_dlcp/source.andro id.com/en//compatibility/ndef-push-protoco l.pdf [retrieved on 2015-01-20]
- "Health Informatics-Personal Health Device Communication Part 20601: Application ProfileOptimized Exchange Protocol;IEEE Std 11073-20601-2008", IEEE STANDARD, IEEE, PISCATAWAY, NJ, USA, 19 December 2008 (2008-12-19), pages c1-198, XP017604162, ISBN: 978-0-7381-5826-6
- BRAVO J ET AL: "Supporting Clinical Information Management by NFC Technology", IFMBE PROCEEDINGS. 4TH EUROPEAN CONFERENCE OF THE INTERNATIONAL FEDERATION FOR MEDICAL AND BIOLOGICAL ENGINEERING; ECIFMBE 2008; 23 - 27 NOVEMBER 2008, ANTWERP, BELGIUM; [IFMBE PROCEEDINGS], [BERLIN] : SPRINGER, DE, 23 November 2008 (2008-11-23), pages 1734-1737, XP009136887, ISBN: 978-3-540-89207-6
- "Simple NDEF Exchange Protocol - Technical Specification, SNEP 1.0", , 31 August 2011 (2011-08-31), pages 1-20, XP055081363, Wakefield, MA, USA 01880 Retrieved from the Internet: URL:http://www.jortec.neec-fct.org/wp-cont ent/uploads/2013/02/Simple_NDEF_Exchange_P rotocol.pdf [retrieved on 2013-09-27]

## Description

### [Technical Field]

The present invention relates to the field of near field communication system, and more particularly relates to a method and system of communicating data in a near field communication environment.

### [Background Art]

Near Field Communication (NFC) is used in devices for communicating with other devices in a network range of less than 10cm. Typically, in NFC ecosystem, user applications can read or write information from or to NFC tags. NFC tags are static in nature and do not have capabilities for dynamically processing of data stored in it. Generally, NFC tags are powered by radio frequency field generated by an active NFC device and are able to respond to requests from the active NFC device.

ISO/IEEE 11073 standards enable communication between medical devices and external systems. Personal health devices which are complaint with ISO/IEEE 11073 standards can communicate with each other using ISO/IEEE 11073-20601 communication protocol. ISO/IEEE 11073 standard defines 'agent' as a node that collects and transmits personal health data to an associated NFC manager and 'manager' as a node receiving personal health data from one or more agents. Exemplary manager includes cell phones, health appliance, set top box, personal computer system and the like. ISO/IEEE 11073-20601 standard defines communication protocol between IEEE 11073 Agent and IEEE 11073 Manager.

Typically, a NFC manager (i.e., IEEE 11073 Manager with a NFC Read/Write Interface) and a NFC agent (i.e., IEEE 11073 Agent with a NFC Tag) communicate in a NFC Reader/Writer mode using a NFC Data Exchange Format (NDEF) message. NDEF message can be of type, text, URI, image, MIME type, etc. In NFC reader/writer mode of communication, NDEF messages are exchanged between the NFC Manager and NFC Agent using the tag transport protocols.

Generally, the ISO/IEEE 11073 Agent and Manager exchange a sequence of ISO/IEEE 11073-20601 APDUs for association, configuration and exchanging measurement data. This involves series of request and response exchanges between the Agent and the Manager.

The Internet-published document "Android NDEF Push Protocol Specification (version 1)", dated 22.2.2011 discloses an NDEF Push Protocol (NPP), which is a protocol to push an NDEF message from a client on one device to a server on another device.

### [Disclosure]

### [Technical Problem]

Usually, when a NFC manager writes a request into a NFC tag residing in an NFC agent, the NFC agent reads the request stored in the NFC tag and writes a response to the request into the NFC tag. The NFC manager reads the response written by the NFC agent from the NFC tag. However, in case the NFC agent delays reading the request from the NFC tag, the NFC manager reads the request written by itself and considers the request as a response from the NFC agent due to limited capabilities of the NFC tag, leading to connection set up latency and communication overhead.

### [Technical Solution]

Aspects of the present invention are to address the above-mentioned problems and/or disadvantages and to provide at least the advantages described below.

The invention is defined by the independent claims. Further embodiments are defined by the dependent claims.

### [Advantageous Effects]

The present invention is to provides a method and system for communicating data in a near field communication (NFC) environment.

### [Description of Drawings]

Figure 1 is a block diagram of a near field communication (NFC) system enabling communication of personal health data between a NFC manager and an NFC agent, according to one embodiment.
Figure 2 is a process flowchart illustrating an exemplary method of reading/writing NFC data exchange format (NDEF) record into the NFC tag, according to one embodiment.
Figure 3a is a schematic representation of a NDEF record, according to one embodiment.
Figure 3b is a schematic representation of a payload field in the NDEF record, according to one embodiment.
Figure 4 is a schematic representation of a payload field in the NDEF record, according to another embodiment.
Figure 5 illustrates a block diagram of the NFC manager showing various components for implementing embodiments of the present subject matter.
Figure 6 illustrates a block diagram of the NFC agent showing various components for implementing embodiments of the present subject matter.

The drawings described herein are for illustration purposes only and are not intended to limit the scope of the present disclosure in any way.

### [Best Mode]

The present invention provides a method and apparatus for communicating personal health data in a near field communication (NFC) environment. In the following detailed description of the embodiments of the invention, reference is made to the accompanying drawings that form a part hereof, and in which are shown by way of illustration specific embodiments in which the invention may be practiced. These embodiments are described in sufficient detail to enable those skilled in the art to practice the invention, and it is to be understood that other embodiments may be utilized and that changes may be made without departing from the scope of the present invention. The following detailed description is, therefore, not to be taken in a limiting sense, and the scope of the present invention is defined only by the appended claims.

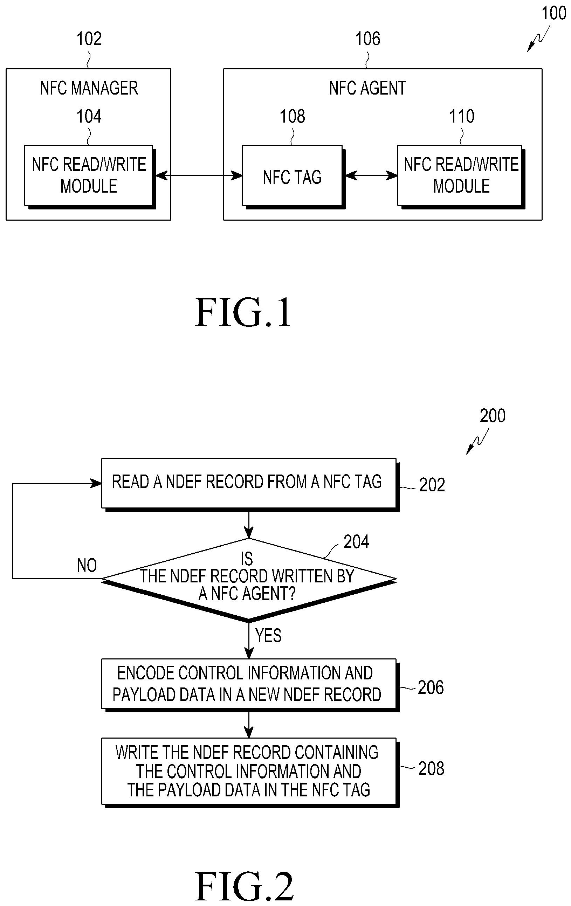

Figure 1 is a block diagram 100 of a near field communication (NFC) system enabling communication of personal health data between a NFC manager 102 and an NFC agent 106, according to one embodiment. Particularly, the NFC system 100 includes the NFC manager 102 having a NFC read/write module 104, and the NFC agent 106 with a NFC tag 108 and a NFC read/write module 110.

The NFC manager 102 may be a device, such as a cell phone, tablet, smart phone, personal digital assistant, set-top box, personal computer and the like, capable of communicating with the NFC agent 106. The NFC agent 106 is a device capable of collecting personal health data and communicating the personal health data with the NFC manager 102 via the NFC tag 108. The NFC manager 102 and the NFC agent 106 writes or reads data in/from the NFC tag 108 using ISO/IEEE 11073-20601 communication protocol.

For communicating personal health data, the NFC manager 102 and the NFC agent 106 exchanges a series of request/response messages via the NFC tag 108. In an exemplary operation, the NFC manager 102 sets control information in a NDEF (NFC data exchange format) record. The NDEF record may contain a header and a payload field. The payload field includes the control information and/or other payload data. It can be noted that, the control information can be encoded in other fields of the NDEF record other than the payload field. The control information may include a direction flag, a state flag, a request/response flag, and a sequence identifier. The direction flag indicates direction of communication of the NDEF record. The state flag indicates state of communication of the NFC manager 102 or the NFC agent 106. The request/response flag indicates whether the NDEF record carries an ISO/IEEE 11073-20601 protocol request command or an ISO/IEEE 11073-20601 protocol response command. The sequence identifier indicates an identifier of NDEF records exchanged between the NFC manager 102 and the NFC agent 106. According to the present invention, the control information is set in the NDEF record to provide synchronized communication between the NFC manager 102 and the NFC agent 106 in order to efficiently obtain personal health data from the NFC agent 106. Upon setting the control information, the NFC read/write module 104 writes the NDEF record containing the control information and/or other payload data into the NFC tag 108.

Subsequently, the NFC read/write module 110 of the NFC agent 106 reads the NDEF record stored in the NFC tag 108. The NFC read/write module 110 retrieves the control information from the NDEF record and determines direction of communication, request/response command, a communication state, and a sequence identifier based on the direction flag, the request/response flag, the state flag, and the sequence identifier. In the above case, the direction flag indicates direction of communication is from the NFC manger 102 to the NFC agent 106. The request/response flag indicates that the NDEF record carries the request command in the payload field. The state flag indicates the communication state of the NFC manager when the NDEF record is written in the NFC tag 108. The sequence identifier indicates the sequence identifier allocated to the NDEF record by the NFC agent. Accordingly, the NFC read/write module 110 updates the control information (i.e., the direction flag, the sequence identifier, the state flag and the request/response flag) in the NDEF record and encodes payload data in the payload field of the NDEF record. Then, the NFC read/write module 110 writes the NDEF record in the NFC tag 108.

Substantially simultaneously, the NFC read/write module 104 reads the NDEF record containing the control information and other payload data from the NFC tag 108. The NFC read/write module 104 then retrieves the control information from the NDEF record and determines direction of communication, request/response command, a communication state, and a sequence identifier based on the direction flag, the request/response flag, the state flag, and the sequence identifier. Accordingly, the NFC read/write module 104 determines whether the NDEF record read from the NFC tag 108 is same as the previous NDEF record written into the NFC tag 108 based on the control information in the read NDEF record. For example, if the direction flag indicates the direction of communication as from the NFC manager 102 to the NFC agent 106 and the request/response flag indicates that the NDEF records contain the request command, then the NFC read/write module 104 identifies the NDEF record as same as the NDEF record previously written by the NFC manager 102 and not as the NDEF record received from the NFC agent 106. In such scenario, the read/write module 104 may ignore the read NDEF record and continue reading the NFC tag 108 after some time interval. It may happen that, the NDEF record read by the NFC read/write module 104 is same as the previously written NDEF record as the NDEF record written in the NFC tag 108 is not yet read by the NFC agent 106 or the NFC read/write module 104 has read the NFC tag 108 prior to writing a NDEF record by the NFC agent 106. In such a case, the control information in the NDEF record enables the NFC manager 102 determine the identity of the NDEF record, according to the present invention.

If the NFC read/write module 104 determines that the NDEF record contains payload data received from the NFC agent 106, then the NFC read/write module 104 processes the payload data and encodes new control information and payload data in a NDEF record. Then, the NFC read/write module 104 writes the NDEF record with the new control information and different payload data in the NFC tag 108. The above process continues till personal health data is communicated to the NFC manager 102 by the NFC agent 106 in the payload field of one of the NDEF records exchanged between the NFC manager 102 and the NFC agent 106.

Figure 2 is a process flowchart 200 illustrating an exemplary method of reading/writing NDEF record into the NFC tag 108, according to one embodiment. At step 202, a NDEF record containing the control information and payload data is read from the NFC tag 108. As described above, the control information includes a direction flag, a state flag, a request/response flag, and a sequence identifier. At step 204, it is determined whether the NDEF record in the NFC tag 108 is written by the NFC agent 106 based on the control information. If the NDEF record is written by the NFC agent 106, then at step 206, the payload data in the read NDEF record is processed and new control information and payload data (e.g., IEEE 11073-20601 data) is encoded in payload field of a new NDEF record. At step 208, the new NDEF record containing the control information and the payload is written in the NFC tag 108. The steps 202-208 are repeated till complete ISO/IEEE 11073 personal health data is received from the NFC agent 106. If the NDEF record is written by the NFC manager 102, then the NDEF record is ignored and the NFC tag 108 is read again after some time to obtain another NDEF record. One skilled in the art will realize that the above steps 202-208 can also be implemented at the NFC agent 106.

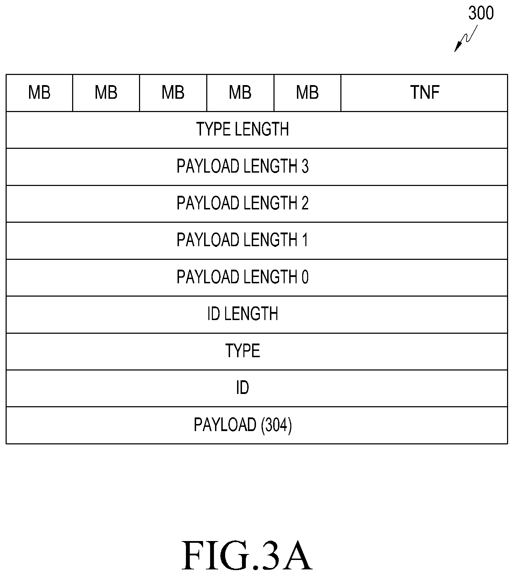

Figure 3a is a schematic representation of a NDEF record 300, according to one embodiment. The NFC record 300 includes a payload field 302. The payload field 302 includes control information associated with the NDEF record and other payload data, according to one embodiment. Alternatively, an ID field 304 includes control information associated with the NDEF record. The NDEF record 300 can be exchanged between the NFC manager 102 and the NFC agent 106 in peer to peer mode using a simple NDEF exchange protocol (SNEP). For example, the NFC manager 102 acting as a SNEP client can exchange the NDEF record 300 with the NFC agent 106 acting as SNEP server. For this, the NFC manager 102 can use SNEP request message with PUT request to send the NDEF record 300 to the NFC agent 106. The NFC agent 106 can send a response NDEF record to the NFC manager 102 using a SNEP response message with PUT request. Alternatively, the NFC manager 102 can use a SNEP request message with a GET request.

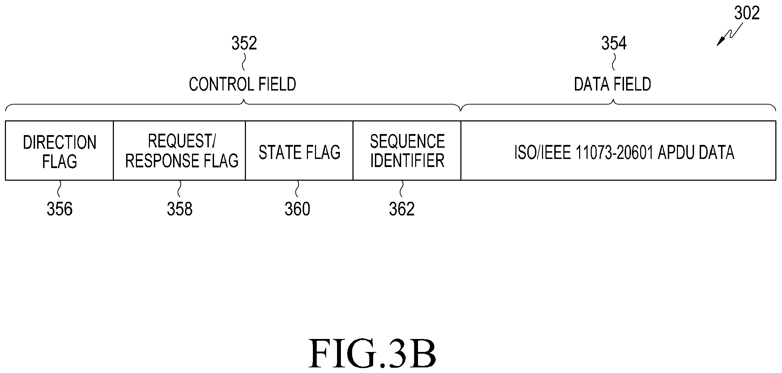

Figure 3b is a schematic representation 350 of the payload field 302 in the NDEF record 300, according to one embodiment. The payload field 302 includes a control field 352 and a data field 354. The control field 352 includes a direction flag field 356, a request/response flag field 358, a state flag field 360, and a sequence identifier field 362. The direction flag field 356 indicates direction of communication of the NDEF record. For example, the direction flag field 356 includes a value '0', if the NDEF record is written into the NFC tag 108 by the NFC agent 106. When the NDEF record is written by the NFC manager 102, the direction flag field 356 includes a value '1'.

The request/response flag field 358 indicates whether the NDEF format includes an ISO/IEEE 11073-20601 communication protocol request command or ISO/IEEE 11073-20601 communication protocol response command. For example, the request/response flag field 358 includes a value '0' when the NDEF record includes a request command and a value '1' when the NDEF record includes a response command. The state flag field 360 indicates a communication state of a sender of the NDEF record. The state flag field 360 helps understand the state of the NFC manager 102 or the NFC agent 106 during a particular instance of communication. Exemplary values carried by the state flag field 360 to indicate a specific state of the NFC manager 102 or the NFC agent 106 is shown in table 1 below:

**[Table 1]**

| | |
|---|---|
| 0x01 | DISCONNECTED |
| 0x01 | CONNECTED |
| 0x02 | UNASSOCIATED |
| 0x03 | ASSOCIATING |
| 0x04 | ASSOCIATED |
| 0x05 | OPERATING |
| 0x06 | CONFIGURING |
| 0x07 | DISASSOCIATING |
| 0x08-0X3F | RFU |

For example, as shown in Table 1, the state flag field 360 may include a value '0x00' when the NFC agent 106 or the NFC manager 102 is in disconnected state. When the NFC agent 106 or the NFC manager 102 is in connected stated, the state flag field 362 will carry a value '0x01'.

The sequence identifier field 362 indicates a sequence identifier assigned to each NDEF record communicated between the NFC read/write module 104/110 and the NFC tag 108. In other words, the sequence identifier field 362 indicates the order in which the NDEF records are written in the NFC tag so that the NDEF records are not duplicated or missed during communication between the NFC manager 102 and the NFC agent 106. The sequence identifier, exchanged between the NFC manager 102 and the NFC agent 106, can be any random number or sequence of numbers incremented by one.

The sequence identifier field 362 enables controlling flow of NDEF records exchanged between the NFC read/write module 104/110 and the NFC tag 108. The sequence identifier in the sequence identifier field 362 provides reliability in communication between the NFC manager 102 and the NFC agent 106. It can be noted that, the sequence field 362 can also be included in a header of the NDEF record. The data field 354 includes ISO/IEEE 11073-20601 data exchanged between the NFC manager 102 and the NFC agent 106.

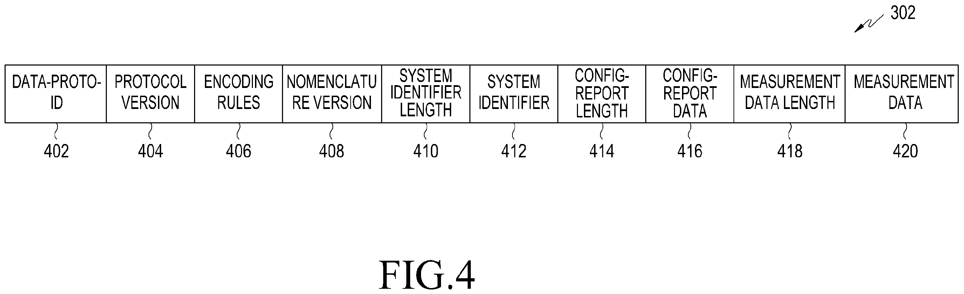

Figure 4 is a schematic representation of the payload field 302 of the NDEF record 300, according to another embodiment. The payload field 302 of Figure 4 is used for communicating measurement data (e.g., physical health data) with a single touch. The payload field 302 encodes complete ISO/IEEE 11073-20601 personal health information, thereby enabling interoperability in exchanging ISO/IEEE 11073 personal health data. The payload field 302 helps reduce connection setup latency and communication overhead.

The payload field 302 includes a data proto-id field 402, a protocol version field 404, an encoding rules field 406, a nomenclature version field 408, a system identifier length field 410, a system identifier field 412, a config-report length field 414, a config-report data field 416, a measurement data length field 418, and a measurement data field 420.

The data proto-id field 402 indicates identifier of data exchange protocol. For example, the data proto-id field 402 includes a value '0', '20601' and '65535'. The value '20601' indicates IEEE 11073-20601 protocol is used. The protocol version field 404 is a one byte field indicating protocol identifier version used by the NFC agent 106. For example, the MSB 4 bits indicate a major release version and LSB 4 bits indicate a minor release version. The encoding rules field 406 indicates specific data APDU encoding rule(s) supported by the NFC agent 106. It is appreciated that, the NFC agent 106 and the NFC manager 102 supports Medical Device Encoding Rules (MDER) and negotiates on other encoding rule(s) except MDER. For example, the encoding rules field 406 includes a value '0' if MDER is supported, a value '1' if Extensible Markup Language Encoding Rules (XER) is supported, and a value '2' if Packed Encoding Rules (PER) is supported.

The nomenclature version field 408 indicates version of nomenclature used as defined in IEEE 11073-20601. For example, the MSB bit is set to a value '0' if the nomenclature version 1 is used. The system identifier length field 410 indicates length of the system identifier field 412. The system identifier field 412 includes a unique system identifier of the NFC agent 106. The EUI-64 format is used to identify the NFC agent 106. The system identifier field 412 enables the NFC manager 102 to determine identity of the NFC agent 106 and to implement a simple access restriction policy.

The config-report length field 414 indicates a length of the configuration report field 416. The conf-report data field 412 carries a configuration event report of the NFC agent 106 starting with object handling bytes. The format of the configuration-report data field 416 is similar to the configuration event report as specified in the ISO/IEEE 11073-20601 specification. The measurement data length 418 indicates length of the measurement data field 420. The measurement data field 420 carries measurement data report from the NFC agent 106 starting with object handle bytes. The format of the measurement data field 420 is similar to the measurement data report as specified in ISO/IEEE 11073-20601 specification.

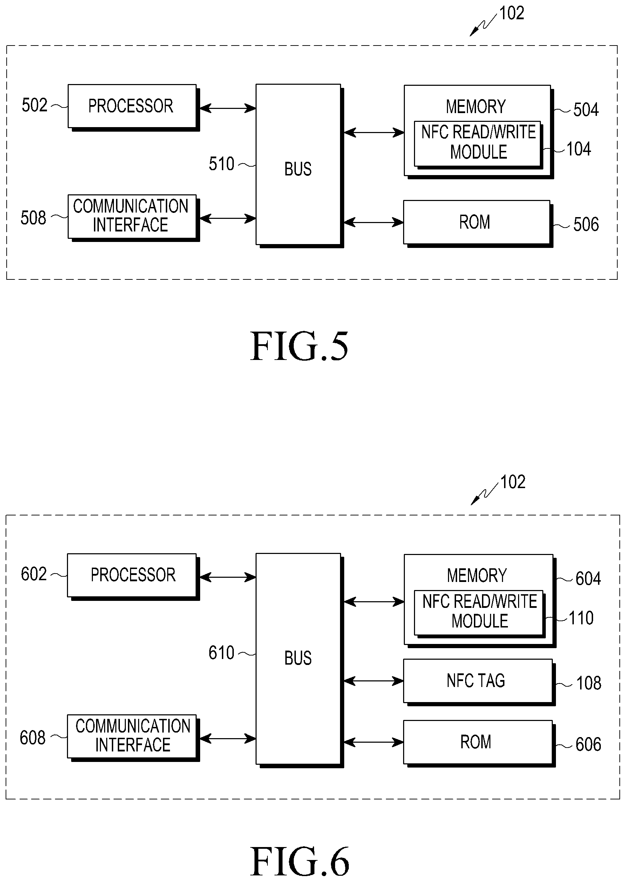

Figure 5 illustrates a block diagram of the NFC manager 102 showing various components for implementing embodiments of the present subject matter. In Figure 5, the NFC manager 102 includes a processor 502, memory 504, a read only memory (ROM) 506, a communication interface 508, and a bus 510.

The processor 502, as used herein, means any type of computational circuit, such as, but not limited to, a microprocessor, a microcontroller, a complex instruction set computing microprocessor, a reduced instruction set computing microprocessor, a very long instruction word microprocessor, an explicitly parallel instruction computing microprocessor, a graphics processor, a digital signal processor, or any other type of processing circuit. The processor 502 may also include embedded controllers, such as generic or programmable logic devices or arrays, application specific integrated circuits, single-chip computers, smart cards, and the like.

The memory 504 may be volatile memory and non-volatile memory. The memory 504 includes the NFC read/write module 104 for reading/writing NDEF records containing control information and other payload data from/to the NFC tag 108, according to the embodiments of the present invention. The communication interface may include 508 may be a radio frequency interface for enabling communication between the NFC Manager 102 and the NFC Agent 106 in a peer-to-peer mode, reader/writer mode, and a card emulation mode. The bus 510 enables communication between the various components of the NFC manager 102 illustrated therein.

Embodiments of the present subject matter may be implemented in conjunction with modules, including functions, procedures, data structures, and application programs, for performing tasks, or defining abstract data types or low-level hardware contexts. Machine-readable instructions stored on any of the above-mentioned storage media may be executable by the processor 502. For example, a computer program may include machine-readable instructions capable of reading/writing NDEF records containing control information and other payload data from/to the NFC tag 108, according to the teachings and herein described embodiments of the present subject matter. In one embodiment, the computer program may be included on a storage medium and loaded from the storage medium to a hard drive in the non-volatile memory.

Figure 6 illustrates a block diagram of the NFC agent 106 showing various components for implementing embodiments of the present subject matter. In Figure 6, the NFC agent 106 includes the NFC tag 108, a processor 602, memory 604, a read only memory (ROM) 606, a communication interface 608, and a bus 610.

The processor 602, as used herein, means any type of computational circuit, such as, but not limited to, a microprocessor, a microcontroller, a complex instruction set computing microprocessor, a reduced instruction set computing microprocessor, a very long instruction word microprocessor, an explicitly parallel instruction computing microprocessor, a graphics processor, a digital signal processor, or any other type of processing circuit. The processor 602 may also include embedded controllers, such as generic or programmable logic devices or arrays, application specific integrated circuits, single-chip computers, smart cards, and the like.

The memory 604 may be volatile memory and non-volatile memory. The memory 604 includes the NFC read/write module 104 for reading/writing NDEF records containing control information and other payload data from/to the NFC tag 108, according to the embodiments of the present invention. The communication interface may include 608 may be a radio frequency interface for enabling communication between the NFC manager102 and the NFC agent 106 in a peer-to-peer mode, reader/writer mode, and a card emulation mode. The bus 610 enables communication between the various components of the NFC agent 106 illustrated therein.

Embodiments of the present subject matter may be implemented in conjunction with modules, including functions, procedures, data structures, and application programs, for performing tasks, or defining abstract data types or low-level hardware contexts. Machine-readable instructions stored on any of the above-mentioned storage media may be executable by the processor 602. For example, a computer program may include machine-readable instructions capable of reading/writing NDEF records containing control information and other payload data from/to the NFC tag 108 and communicating the NDEF records stored in the NFC tag into the NFC manger 102, according to the teachings and herein described embodiments of the present subject matter. In one embodiment, the computer program may be included on a storage medium and loaded from the storage medium to a hard drive in the non-volatile memory.

The present embodiments have been described with reference to specific example embodiments, it will be evident that various modifications and changes may be made to these embodiments without departing from the scope of the various embodiments. Furthermore, the various devices, modules, selectors, estimators, and the like described herein may be enabled and operated using hardware circuitry, for example, complementary metal oxide semiconductor based logic circuitry, firmware, software and/or any combination of hardware, firmware, and/or software embodied in a machine readable medium. For example, the various electrical structure and methods may be embodied using transistors, logic gates, and electrical circuits, such as application specific integrated circuit.

Aspects of the invention can be understood from the following clauses:
[Clause 1] A method of communicating personal health data in a near field communication (NFC) environment, comprising:
   setting control information in a NFC data exchange format (NDEF) record by a first NFC device to synchronize communication between the first NFC device and a second NFC device in a NFC environment, wherein the control information includes at least one of a direction flag, a request/response type flag, a status flag and a sequence identifier; and
   writing the NDEF record containing the control information into a NFC tag.
[Clause 2] The method of clause 1, further comprising:
   reading a NDEF record containing control information from the NFC tag by the first NFC device;
   determining whether the NDEF record is written into the NFC tag by a second NFC device based on the control information in the NDEF record;
   if so, processing remaining payload data in the NDEF record; and
   if not, ignoring the NDEF record read from the NFC tag interface.
[Clause 3] The method of clause 2, further comprising:
   repeating the steps of setting, transmitting, reading, and determining till complete personal health data is communicated.
[Clause 4] The method of clause 3, wherein the NDEF record comprises a payload field carrying the control information and said remaining payload data.
[Clause 5] The method of clause 4, wherein the remaining payload data in the payload field comprises personal health data.
[Clause 6] The method of clause 2, wherein the direction flag indicates whether the NDEF record is written in the NFC tag by the first NFC device or the second NFC device.
[Clause 7] The method of clause 2, wherein the request/response type flag indicates whether said remaining payload data in the payload field is an ISO/IEEE 11073-20601 protocol request command or an ISO/IEEE 11073-20601 protocol response command.
[Clause 8] The method of clause 2, wherein the state flag indicates a state of the first NFC device or the second NFC device while writing the NDEF record in the NFC tag.
[Clause 9] The method of clause 8, wherein the state is selected from the group consisting of disconnected state, connected state, unassociated state, associating state, associated state, operating state, configuring state, and disassociating state.
[Clause 10] The method of clause 2, wherein the sequence identifier indicates a sequence identifier associated with the NDEF record.
[Clause 11] The method of clause 1, wherein the NFC tag resides in one of the first NFC device and the second NFC device.
[Clause 12] An apparatus comprising:
   a processor; and
   memory coupled to the processor, wherein the memory comprises a read/write module configured for:
      setting control information in a NFC data exchange format (NDEF) record, wherein the control information includes a direction flag, a request/response type flag, a status flag and a sequence identifier; and
      writing the NDEF record containing the control information into a NFC tag.
[Clause 13] The apparatus of clause 12, wherein the read/write module is further configured for:
   reading a NDEF record containing control information from the NFC tag;
   determining whether the NDEF record is written into the NFC tag by another NFC device based on the control information in the NDEF record;
   if so, processing remaining payload data in the NDEF record; and
   if not, ignoring payload data in the NDEF record read from the NFC tag.
[Clause 14] The apparatus of clause 13, wherein the NDEF record comprises a payload field carrying the control information and the remaining payload data.
[Clause 15] The apparatus of clause 14, wherein said remaining payload data in the payload field comprises personal health data.
[Clause 16] The apparatus of clause 13, wherein the direction flag indicates direction of communication of the NDEF record.
[Clause 17] The apparatus of clause 13, wherein the request/response type flag indicates whether the remaining payload data in the payload field is an ISO/IEEE 11073-20601 protocol request command or an ISO/IEEE 11073-20601 protocol response command.
[Clause 18] The apparatus of clause 13, wherein the state flag indicates a communication state of sender of the NDEF record.
[Clause 19] The apparatus of clause 18, wherein the state is selected from the group consisting of disconnected state, connected state, unassociated state, associating state, associated state, operating state, configuring state, and disassociating state.
[Clause 20] The apparatus of clause 13, wherein the sequence identifier indicates a sequence identifier associated with the NDEF record.

## Claims

1. A method for transmitting and receiving data by a near field communication, NFC, manager (102) in NFC environment, the method comprising:
setting control information in a NFC Data Exchange Format, NDEF, record;
writing, in a NFC tag (108), the control information of the NDEF record including payload data;
reading changed control information of changed NDEF record including changed payload data from the NFC tag; and
determining whether the changed NDEF record is identical to a NDEF record previously read from the NFC tag,
wherein the determination operation comprises:
reading a sequence identifier included in the changed control information of the changed NDEF record,
when the sequence identifier is even, determining that the changed NDEF record is not identical to the NDEF record previously read from the NFC tag, and
when the sequence identifier is odd, determining that the changed NDEF record is identical to the NDEF record previously read from the NFC tag.

2. The method of claim 1, wherein the control information includes at least one of a request/response flag, a status flag, and sequence identifier.

3. The method of claim 2, wherein determining whether the changed NDEF record is identical to the NDEF record previously read from the NFC tag comprises:
if the sequence identifier is 0, determining that the changed NDEF record is not identical to the NDEF record previously read from the NFC tag.

4. The method of claim 2, wherein the status flag is indicating a status of the NFC communication through the NFC tag.

5. The method of claim 2, wherein,
if the request/response flag is 0, the NDEF record includes an ISO/IEEE 11073-20601 protocol request command, and
if the request/response flag is 1, the NDEF record includes an ISO/IEEE 11073-20601 protocol response command.

6. A near field communication, NFC, manager (102) for transmitting and receiving data in NFC environment, the NFC manager comprising:
a read/write module (104) configured to write NDEF record including control information or payload data in a NFC tag; and
a processor (502) configured to:
set control information in a NDEF record,
control the read/write module to read changed control information of changed NDEF record comprising changed payload data from the NFC tag, and
determine whether the changed NDEF record is identical to a NDEF record previously read from the NFC tag (108),
wherein, in the determination operation, the processor is further configured to:
read a sequence identifier included in the changed control information of the changed NDEF record,
when the sequence identifier is even, determine that the changed NDEF record is not identical to the NDEF record previously read from the NFC tag, and
when the sequence identifier is odd, determine that the changed NDEF record is identical to the NDEF record previously read from the NFC tag.

7. The NFC manager of claim 6, wherein the control information includes at least one of a request/response flag, a status flag, and sequence identifier.

8. The NFC manager of claim 6, wherein,
if the sequence identifier is 0, the processor determines that the changed NDEF record is not identical to the NDEF record previously read from the NFC tag.

9. The NFC manager of claim 6, wherein the status flag is indicating a status of the NFC communication through the NFC tag.

10. The NFC manager of claim 6, wherein,
if the request/response flag is 0, the NDEF record includes an ISO/IEEE 11073-20601 protocol request command, and
if the request/response flag is 1, the NDEF record includes an ISO/IEEE 11073-20601 protocol response command.

11. A method for transmitting and receiving data by a near field communication, NFC, agent (106) in NFC environment, the method comprising:
writing a NDEF record stored in a NFC tag;
changing the NDEF record based on control information set in the NDEF record; and
writing the changed NDEF record in the NFC tag,
wherein changing the NDEF record comprises:
when the sequence identifier is even, determining that the NFC agent does not change the NDEF record, and
when the sequence identifier is odd, determining that the NFC agent changes the NDEF record.

12. The method of claim 11, wherein the control information includes at least one of a request/response flag, a status flag, and sequence identifier.

13. The method of claim 11, wherein determining whether changing the NDEF record based on the control information set in the NDEF record comprises:
changing the control information set in the NDEF record; and
encoding payload data of the NDEF record.

14. The method of claim 12, further comprising:
if the sequence identifier is 0, determining that the NFC agent doesn't change the NDEF record.

15. The method of claim 12, wherein the status flag is indicating a status of the NFC communication through the NFC tag.

16. The method of claim 12, wherein,
if the request/response flag is 0, the NDEF record includes an ISO/IEEE 11073-20601 protocol request command, and
if the request/response flag is 1, the NDEF record includes an ISO/IEEE 11073-20601 protocol response command.

17. A near field communication, NFC, agent (106) for transmitting and receiving data in NFC environment, the NFC agent comprising:
a read/write module (110) configured to write a NDEF record stored in a NFC tag (108); and
a processor (602) configured to:
change the NDEF record based on control information set in the NDEF record, and
control the read/write module to write the changed NDEF record in the NFC tag,
wherein the processor is further configured to:
determine that the NFC agent does not change the NDEF record when the sequence identifier is even, and
determine that the NFC agent changes the NDEF record when the sequence identifier is odd.

18. The NFC agent of claim 17, wherein the control information includes at least one of a request/response flag, a status flag, and sequence identifier.

19. The NFC agent of claim 17, wherein,
the processor changes the NDEF message based on the control information by changing the control information set in the NDEF record, and encoding payload data of the NDEF record.

20. The NFC agent of claim 18, wherein,
if the sequence identifier is 0, the processor determines that the NFC agent doesn't change the NDEF record.

21. The NFC agent of claim 18, wherein the status flag is indicating a status f the NFC communication through the NFC tag.

22. The NFC agent of claim 18, wherein,
if the request/response flag is 0, the NDEF record includes an ISO/IEEE 11073-20601 protocol request command, and
if the request/response flag is 1, the NDEF record includes an ISO/IEEE 11073-20601 protocol response command.

## Patentansprüche

1. Verfahren zum Übertragen und Empfangen von Daten durch einen Nahfeldkommunikations(NFC)-Manager (102) in einer NFC-Umgebung, wobei das Verfahren Folgendes umfasst:
Festlegen von Steuerinformationen in einem NFC Data Exchange Format(NDEF)-Datensatz;
Schreiben in einem NFC-Tag (108) der Steuerinformationen des NDEF-Datensatzes, einschließlich Nutzdaten;
Lesen von geänderten Steuerinformationen von geänderten NDEF-Datensätzen, einschließlich geänderter Nutzdaten von dem NFC-Tag; und
Bestimmen, ob der geänderte NDEF-Datensatz identisch mit einem NDEF-Datensatz ist, der zuvor von dem NFC-Tag gelesen wurde,
wobei die Ermittlungsoperation Folgendes umfasst:
Lesen einer Sequenzkennung, die in den geänderten Steuerinformationen des geänderten NDEF-Datensatzes eingeschlossen ist,
wenn die Sequenzkennung gerade ist, Bestimmen, dass der geänderte NDEF-Datensatz nicht identisch mit dem NDEF-Datensatz ist, der zuvor von dem NFC-Tag gelesen wurde, und
wenn die Sequenzkennung ungerade ist, Bestimmen, dass der geänderte NDEF-Datensatz identisch mit dem NDEF-Datensatz ist, der zuvor von dem NFC-Tag gelesen wurde.

2. Verfahren nach Anspruch 1, wobei die Steuerinformationen mindestens ein Anfrage-/Antwort-Flag, ein Status-Flag oder eine Sequenzkennung einschließen.

3. Verfahren nach Anspruch 2, wobei das Bestimmen, ob der geänderte NDEF-Datensatz identisch mit dem NDEF-Datensatz ist, der zuvor von dem NFC-Tag gelesen wurde, Folgendes umfasst:
wenn die Sequenzkennung 0 ist, Bestimmen, dass der geänderte NDEF-Datensatz nicht identisch mit dem NDEF-Datensatz ist, der zuvor von dem NFC-Tag gelesen wurde.

4. Verfahren nach Anspruch 2, wobei das Status-Flag einen Status der NFC-Verbindung über das NFC-Flag anzeigt.

5. Verfahren nach Anspruch 2, wobei,
wenn das Anfrage-/Antwort-Flag 0 ist, der NDEF-Datensatz einen Protokollanfragebefehl gemäß ISO/IEEE 11073-20601 einschließt, und
wenn das Anfrage-/Antwort-Flag 1 ist, der NDEF-Datensatz einen Protokollantwortbefehl gemäß ISO/IEEE 11073-20601 einschließt.

6. Nahfeldkommunikations(NFC)-Manager (102) zum Übertragen und Empfangen von Daten in NFC-Umgebung, wobei der NFC-Manager Folgendes umfasst:
ein Lese-/Schreibmodul (104), konfiguriert, um NDEF-Datensätze, einschließlich Steuerinformationen oder Nutzdaten, in ein NFC-Tag zu schreiben; und
einen Prozessor (502), der für Folgendes konfiguriert ist:
Festlegen von Steuerinformationen in einen NDEF-Datensatz,
Steuern des Lese-/Schreibmoduls, um geänderte Steuerinformationen von geänderten NDEF-Datensätzen mit geänderten Nutzdaten von dem NFC-Tag zu lesen, und
Bestimmen, ob der geänderte NDEF-Datensatz identisch mit einem NDEF-Datensatz ist, der zuvor von dem NFC-Tag (108) gelesen wurde,
wobei bei der Ermittlungsoperation der Prozessor ferner für Folgendes konfiguriert ist:
Lesen einer Sequenzkennung, die in den geänderten Steuerinformationen des geänderten NDEF-Datensatzes eingeschlossen ist,
wenn die Sequenzkennung gerade ist, Bestimmen, dass der geänderte NDEF-Datensatz nicht identisch mit dem NDEF-Datensatz ist, der zuvor von dem NFC-Tag gelesen wurde, und
wenn die Sequenzkennung ungerade ist, Bestimmen, dass der geänderte NDEF-Datensatz identisch mit dem NDEF-Datensatz ist, der zuvor von dem NFC-Tag gelesen wurde.

7. NFC-Manager nach Anspruch 6, wobei die Steuerinformationen mindestens ein Anfrage-/Antwort-Flag, ein Status-Flag oder eine Sequenzkennung einschließen.

8. NFC-Manager nach Anspruch 6, wobei,
wenn die Sequenzkennung 0 ist, der Prozessor bestimmt, dass der geänderte NDEF-Datensatz nicht identisch mit dem NDEF-Datensatz ist, der vorher von dem NFC-Tag gelesen wurde.

9. NFC-Manager nach Anspruch 6, wobei das Status-Flag einen Status der NFC-Verbindung über das NFC-Tag anzeigt.

10. NFC-Manager nach Anspruch 6, wobei,
wenn das Anfrage-/Antwort-Flag 0 ist, der NDEF-Datensatz einen Protokollanfragebefehl gemäß ISO/IEEE 11073-20601 einschließt, und
wenn das Anfrage-/Antwort-Flag 1 ist, der NDEF-Datensatz einen Protokollantwortbefehl gemäß ISO/IEEE 11073-20601 einschließt.

11. Verfahren zum Übertragen und Empfangen von Daten durch einen Nahfeldkommunikations(NFC)-Agenten (106) in einer NFC-Umgebung, wobei das Verfahren Folgendes umfasst:
Schreiben eines NDEF-Datensatzes, der in einem NFC-Tag gespeichert ist;
Ändern des NDEF-Datensatzes basierend auf Steuerinformationen, die in dem NDEF-Datensatz festgelegt sind; und
Schreiben des geänderten NDEF-Datensatzes in dem NFC-Tag,
wobei das Ändern des NDEF-Datensatzes Folgendes umfasst:
wenn die Sequenzkennung gerade ist, Bestimmen, ob der NFC-Agent den NDEF-Datensatz nicht ändert, und
wenn die Sequenzkennung ungerade ist, Bestimmen, ob der NFC-Agent den NDEF-Datensatz ändert.

12. Verfahren nach Anspruch 11, wobei die Steuerinformationen mindestens ein Anfrage-/Antwort-Flag, ein Status-Flag oder eine Sequenzkennung einschließen.

13. Verfahren nach Anspruch 11, wobei das Bestimmen, ob das Ändern des NDEF-Datensatzes basierend auf den Steuerinformationen, die in dem NDEF-Datensatz festgelegt sind, Folgendes umfasst:
Ändern der Steuerinformationen, die in dem NDEF-Datensatz festgelegt sind; und
Codierung von Nutzdaten des NDEF-Datensatzes.

14. Verfahren nach Anspruch 12, das ferner Folgendes umfasst:
wenn die Sequenzkennung 0 ist, Bestimmen, dass der NFC-Agent nicht den NDEF-Datensatz ändert.

15. Verfahren nach Anspruch 12, wobei das Status-Flag einen Status der NFC-Verbindung über das NFC-Tag anzeigt.

16. Verfahren nach Anspruch 12, wobei,
wenn das Anfrage-/Antwort-Flag 0 ist, der NDEF-Datensatz einen Protokollanfragebefehl gemäß ISO/IEEE 11073-20601 einschließt, und
wenn das Anfrage-/Antwort-Flag 1 ist, der NDEF-Datensatz einen Protokollantwortbefehl gemäß ISO/IEEE 11073-20601 einschließt.

17. Nahfeldkommunikations(NFC)-Agent (106) zum Übertragen und Empfangen von Daten in einer NFC-Umgebung, wobei der NFC-Agent Folgendes umfasst:
ein Lese-/Schreib-Modul (110), konfiguriert, um einen NDEF-Datensatz zu schreiben, der in einem NFC-Tag (108) gespeichert ist; und
einen Prozessor (602), der für Folgendes konfiguriert ist:
Ändern des NDEF-Datensatzes basierend auf Steuerinformationen, die in dem NDEF-Datensatz festgelegt sind, und
Steuern des Lese-/Schreib-Moduls, um den geänderten NDEF-Datensatz in dem NFC-Tag zu schreiben, wobei der Prozessor ferner für Folgendes konfiguriert ist:
Bestimmen, dass der NFC-Agent nicht den NDEF-Datensatz ändert, wenn die Sequenzkennung gerade ist, und
Bestimmen, dass der NFC-Agent den NDEF-Datensatz ändert, wenn die Sequenzkennung ungerade ist.

18. NFC-Agent nach Anspruch 17, wobei die Steuerinformationen mindestens ein Anfrage-/Antwort-Flag, ein Status-Flag oder eine Sequenzkennung einschließen.

19. NFC-Agent nach Anspruch 17, wobei
der Prozessor die NDEF-Nachricht basierend auf den Steuerinformationen ändert, indem er die Steuerinformationen, die in dem NDEF-Datensatz festgelegt sind, ändert und Nutzdaten des NDEF-Datensatzes codiert.

20. NFC-Agent nach Anspruch 18, wobei,
wenn die Sequenzkennung 0 ist, der Prozessor bestimmt, dass der NFC-Agent nicht den NDEF-Datensatz ändert.

21. NFC-Agent nach Anspruch 18, wobei das Status-Flag einen Status der NFC-Verbindung über das NFC-Tag anzeigt.

22. NFC-Agent nach Anspruch 18, wobei,
wenn das Anfrage-/Antwort-Flag 0 ist, der NDEF-Datensatz einen Protokollanfragebefehl gemäß ISO/IEEE 11073-20601 einschließt, und
wenn das Anfrage-/Antwort-Flag 1 ist, der NDEF-Datensatz einen Protokollantwortbefehl gemäß ISO/IEEE 11073-20601 einschließt.

## Revendications

1. Procédé pour transmettre et recevoir des données au moyen d'un gestionnaire (102) de communication en champ proche, NFC, dans un environnement de NFC, le procédé comprenant :
définir des informations de contrôle dans un enregistrement en format d'échange de données de communication en champ proche NFC, NDEF ;
écrire, sur une étiquette de NFC (108), les informations de contrôle de l'enregistrement en NDEF comprenant des données de charge utile ;
lire des informations de contrôle modifiées de l'enregistrement en NDEF modifié comprenant les données de charge utile modifiées figurant sur l'étiquette de NFC ; et
déterminer si l'enregistrement en NDEF modifié est identique à un enregistrement en NDEF lu précédemment sur l'étiquette de NFC,
où l'opération de détermination comprend :
lire un identifiant de séquence inclus dans les informations de contrôle modifiées sur l'enregistrement en NDEF modifié,
lorsque l'identifiant de séquence est pair, déterminer que l'enregistrement en NDEF modifié n'est pas identique à l'enregistrement en NDEF lu précédemment sur l'étiquette de NFC, et
lorsque l'identifiant de séquence est impair, déterminer que l'enregistrement en NDEF modifié est identique à l'enregistrement en NDEF lu précédemment sur l'étiquette de NFC.

2. Procédé selon la revendication 1, où les informations de contrôle comprennent au moins un parmi un indicateur de demande / réponse, un indicateur d'état et un identifiant de séquence.

3. Procédé selon la revendication 2, où déterminer si l'enregistrement en NDEF modifié est identique à l'enregistrement en NDEF lu précédemment sur l'étiquette de NFC comprend :
si l'identifiant de séquence est 0, déterminer que l'enregistrement en NDEF modifié n'est pas identique à l'enregistrement en NDEF lu précédemment sur l'étiquette de NFC.

4. Procédé selon la revendication 2, où l'indicateur d'état indique un état de la communication de NFC par l'intermédiaire de l'étiquette de NFC.

5. Procédé selon la revendication 2, où,
si l'indicateur de demande / réponse est 0, l'enregistrement en NDEF comprend une commande de demande de protocole ISO/IEEE 11073-20601, et
si l'indicateur de demande / réponse est 1, l'enregistrement en NDEF comprend une commande de réponse de protocole ISO/IEEE 11073-20601.

6. Gestionnaire (102) de communication en champ proche, NFC, destiné à transmettre et à recevoir des données dans un environnement de NFC, le gestionnaire de NFC comprenant :
un module de lecture / écriture (104) configuré pour écrire un enregistrement en NDEF incluant des informations de contrôle ou des données de charge utile sur une étiquette de NFC ; et
un processeur (502) configuré pour :
définir des informations de contrôle dans un enregistrement en NDEF,
contrôler le module de lecture / écriture pour lire des informations de contrôle modifiées de l'enregistrement en NDEF modifié comprenant des données de charge utile modifiées de l'étiquette de NFC, et
déterminer si l'enregistrement en NDEF modifié est identique à un enregistrement en NDEF lu précédemment sur l'étiquette de NFC (108),
où, dans l'opération de détermination, le processeur est, en outre, configuré pour :
lire un identifiant de séquence inclus dans les informations de contrôle modifiées de l'enregistrement en NDEF modifié,
lorsque l'identifiant de séquence est pair, déterminer que l'enregistrement en NDEF modifié n'est pas identique à l'enregistrement en NDEF lu précédemment sur l'étiquette de NFC, et
lorsque l'identifiant de séquence est impair, déterminer que l'enregistrement en NDEF modifié est identique à l'enregistrement en NDEF lu précédemment sur l'étiquette de NFC.

7. Gestionnaire de NFC selon la revendication 6, où les informations de contrôle comprennent au moins un parmi un indicateur de demande / réponse, un indicateur d'état et un identifiant de séquence.

8. Gestionnaire de NFC selon la revendication 6, où,
si l'identifiant de séquence est 0, le processeur détermine que l'enregistrement en NDEF modifié n'est pas identique à l'enregistrement en NDEF lu précédemment sur l'étiquette de NFC.

9. Gestionnaire de NFC selon la revendication 6, où l'indicateur d'état indique un état de la communication de NFC par l'intermédiaire de l'etiquette de NFC.

10. Gestionnaire de NFC selon la revendication 6, où,
si l'indicateur de demande / réponse est 0, l'enregistrement en NDEF comprend une commande de demande de protocole ISO/IEEE 11073-20601, et
si l'indicateur de demande / réponse est 1, l'enregistrement en NDEF comprend une commande de réponse de protocole ISO/IEEE 11073-20601.

11. Procédé de transmission et de réception de données par un agent de communication en champ proche, NFC, (106) dans un environnement de NFC, le procédé comprenant :
écrire un enregistrement en NDEF stocké dans une étiquette de NFC ;
modifier l'enregistrement en NDEF en fonction des informations de contrôle définies dans l'enregistrement en NDEF ; et
écrire l'enregistrement en NDEF modifié dans l'étiquette de NFC,
où la modification de l'enregistrement en NDEF comprend :
lorsque l'identifiant de séquence est pair, déterminer que l'agent de NFC ne modifie pas l'enregistrement en NDEF, et
lorsque l'identifiant de séquence est impair, déterminer que l'agent de NFC modifie l'enregistrement en NDEF.

12. Procédé selon la revendication 11, où les informations de contrôle comprennent au moins un d'un indicateur de demande / réponse, un indicateur d'état et un identifiant de séquence.

13. Procédé selon la revendication 11, où déterminer s'il convient de modifier l'enregistrement en NDEF en fonction des informations de contrôle définies dans l'enregistrement en NDEF comprend :
modifier les informations de contrôle définies dans l'enregistrement en NDEF ; et
coder des données de charge utile de l'enregistrement en NDEF.

14. Procédé selon la revendication 12, comprenant en outre :
si l'identifiant de séquence est 0, déterminer que l'agent de NFC ne modifie pas l'enregistrement en NDEF.

15. Procédé selon la revendication 12, où l'indicateur d'état indique un état de la communication de NFC par l'intermédiaire de l'étiquette de NFC.

16. Procédé selon la revendication 12, où,
si l'indicateur de demande / réponse est 0, l'enregistrement en NDEF comprend une commande de demande de protocole ISO/IEEE 11073-20601, et
si l'indicateur de demande / réponse est 1, l'enregistrement en NDEF comprend une commande de réponse de protocole ISO/IEEE 11073-20601.

17. Agent (106) de communication en champ proche, NFC, pour transmettre et recevoir des données dans un environnement de NFC, l'agent de NFC comprenant :
un module de lecture / écriture (110) configuré pour écrire un enregistrement en NDEF stocké dans une étiquette de NFC (108) ; et
un processeur (602) configuré pour :
modifier l'enregistrement en NDEF en fonction des informations de contrôle définies dans l'enregistrement en NDEF, et
contrôler le module de lecture / écriture pour écrire l'enregistrement en NDEF modifié dans l'étiquette de NFC, où le processeur est, en outre, configuré pour :
déterminer que l'agent de NFC ne modifie pas l'enregistrement en NDEF lorsque l'identifiant de séquence est pair, et
déterminer que l'agent de NFC modifie l'enregistrement en NDEF lorsque l'identifiant de séquence est impair.

18. Agent de NFC selon la revendication 17, où les informations de contrôle comprennent au moins un d'un indicateur de demande / réponse, un indicateur d'état et un identifiant de séquence.

19. Agent de NFC selon la revendication 17, où,
le processeur modifie le message en NDEF en fonction des informations de contrôle en modifiant les informations de contrôle définies dans l'enregistrement en NDEF et en codant les données de charge utile de l'enregistrement en NDEF.

20. Agent de NFC selon la revendication 18, où,
si l'identifiant de séquence est 0, le processeur détermine que l'agent de NFC ne modifie pas l'enregistrement en NDEF.

21. Agent de NFC selon la revendication 18, où l'indicateur d'état indique un état de la communication de NFC par l'intermédiaire de l'étiquette de NFC.

22. Agent de NFC selon la revendication 18, où,
si l'indicateur de demande / réponse est 0, l'enregistrement en NDEF comprend une commande de demande de protocole ISO/IEEE 11073-20601, et
si l'indicateur de demande / réponse est 1, l'enregistrement en NDEF comprend une commande de réponse de protocole ISO/IEEE 11073-20601.
